# EUROPÄISCHE PATENTSCHRIFT

(11) **EP 2 376 133 B1**
(45) Veröffentlichungstag und Bekanntmachung des Hinweises auf die Patenterteilung: **31.10.2012**
(21) Anmeldenummer: 09760773.3
(22) Anmeldetag: 28.11.2009
(51) Int. Cl.: A61L 24/04, C08G 18/10, C09J 175/02, C09J 175/04

(54) **MEDIZINISCHE KLEBSTOFFE FÜR DIE CHIRURGIE**
MEDICAL ADHESIVE FOR SURGERY
COLLE MÉDICALE UTILISÉE EN CHIRURGIE

(30) Priorität: 12.12.2008 EP 08021576
(43) Veröffentlichungstag der Anmeldung: 19.10.2011
(73) Patentinhaber: Bayer MaterialScience AG, 51368 Leverkusen (DE)
(72) Erfinder: HECKROTH, Heike, 51519 Odenthal (DE); KÖHLER, Burkhard, 34289 Zierenberg (DE)
(86) Internationale Anmeldenummer: PCT/EP2009/008498
(87) Internationale Veröffentlichungsnummer: WO 2010/066356

(56) Entgegenhaltungen:
- EP-A- 1 081 171
- EP-A- 1 719 530
- DE-A1- 10 242 075
- US-A1- 2004 067 315
- 'Medical Devices: Guidance document', [Online] 03 Dezember 2009, XP055023202 Gefunden im Internet: <URL:http://ec.europa.eu/health/medical-dev ices/files/meddev/2_1_3_rev_3-12_2009_en.pd f> [gefunden am 2012-03-28]

## Beschreibung

Die vorliegende Erfindung betrifft neuartige, schnell härtende Klebstoffe auf Basis hydrophiler Polyisocyanat-Prepolymere für den Einsatz in der Chirurgie.

In den vergangenen Jahren hat sich ein steigendes Interesse entwickelt, chirurgische Nähte durch den Einsatz von geeigneten Klebstoffen zu ersetzen oder zu unterstützen. Besonders im Bereich der plastischen Chirurgie, in der auf dünne, möglichst unsichtbare Narben Wert gelegt wird, werden vermehrt Klebstoffe eingesetzt.

Gewebekleber müssen eine Reihe von Eigenschaften haben, um bei den Chirurgen als Nahtersatz akzeptiert zu werden. Hierzu gehören eine leichte Verarbeitbarkeit und eine Eingangsviskosität, so dass der Klebstoff nicht in tiefere Gewebeschichten eindringen oder verlaufen kann. In der klassischen Chirurgie wird eine schnelle Aushärtung erfordert, wogegen in der plastischen Chirurgie eine Korrektur der Klebenaht möglich sein sollte und damit die Aushärtungsgeschwindigkeit nicht zu schnell sein darf (ca. 1-5 min). Die Klebeschicht sollte ein flexibler, transparenter Film sein, der nicht in einem Zeitraum kleiner drei Wochen abgebaut wird. Der Klebstoff muß biokompatibel sein und darf weder Histotoxicität noch Thrombogenität oder ein allergenes Potential besitzen.

Diverse Materialien, die als Gewebekleber eingesetzt werden, sind im Handel erhältlich. Hierzu gehören die Cyanacrylate Dernabond® (Octyl-2-Cyanoacrylat) und Histoacryl Blue® (Butyl-Cyanoacrylat). Die schnelle Aushärtezeit, sowie die Sprödigkeit der Klebestelle limitieren jedoch den Einsatz. Durch die schlechte Bioabbaubarkeit sind Cyanacrylate nur für äußerliche chirurgische Nähte geeignet.

Als Alternative zu den Cyanacrylaten stehen biologische Klebstoffe wie peptidbasierte Substanzen (BioGlue^{®}) oder Fibrinkleber (Tissucol) zur Verfügung. Neben den hohen Kosten zeichnen sich Fibrinkleber durch eine relative schwache Klebstärke und einen schnellen Abbau aus, so dass dieser nur bei kleineren Schnitten auf nicht gespannter Haut anwendbar ist.

Isocyanat-haltige Klebstoffe basieren alle auf einem aromatischen Diisocyanat und einem hydrophilen Polyol, wobei bevorzugt die Isocyanate TDI und MDI eingesetzt werden (US 20030135238, US 20050129733). Beide können, um die Reaktivität zu erhöhen, Elektronen ziehende Substituenten tragen (WO-A 03/9323).

Schwierigkeiten waren bislang die geringe mechanische Stärke (US 5,156,613), zu langsame Aushärtungsgeschwindigkeit (US 4,806,614), die zu schnelle Bioabbaubarkeit (US 6,123,667) sowie unkontrollierte Schwellung (US 6,265,016).

Gemäß Patent US 20030135238 stellen ausschließlich Polyurethan-Prepolymere mit trifunktioneller oder verzweigter Struktur, die dazu in der Lage sind, Hydrogele zu bilden, geeignete Klebstoffe dar. Der Klebstoff muß dabei in der Lage sein, eine kovalente Bindung zum Gewebe auszubilden. US 20030135238 und US 20050129733 beschreiben die Synthese trifunktioneller, Ethylenoxid reicher TDI-und IPDI- (US 20030135238) basierter Prepolymere, die mit Wasser oder mit Gewebeflüssigkeiten zum Hydrogel reagieren. Ausreichend schnelle Aushärtung wurde bislang nur bei Verwendung aromatischer Isocyanate erreicht, welche jedoch unter Schaumbildung reagieren. Dadurch kommt es zu einem Eindringen des Klebstoffes in die Wunde und damit zum Auseinanderdrücken der Wundränder, was eine schlechtere Heilung mit verstärkter Narbenbildung mit sich bringt. Durch die Schaumbildung ist zudem die mechanische Festigkeit sowie die Haftung der Klebeschicht herabgesetzt. Aufgrund der hohen Reaktivität der Prepolymere kommt es zudem zu einer Reaktion der Isocyanatreste mit dem Gewebe, wobei oftmals eine Denaturierung, erkennbar durch Weißfärbung des Gewebes, auftritt.

Als Ersatz der aromatischen Isocyanate wurde Lysindiisocyanat untersucht, welches jedoch aufgrund seiner geringen Reaktivität nicht oder nur langsam mit Gewebe reagiert (US 20030135238).

Aliphatische Isocyanate wurden, um die Reaktivität zu erhöhen, fluoriert (US 5,173,301), wodurch es jedoch spontan zu einer Selbstpolymerisation des Isocyanates kam.

EP-A 0 482 467 beschreibt die Synthese eines chirurgischen Klebstoffes basierend auf einem aliphatischen Isocyanat (bevorzugt HDI) und einem Polyethylenglycol (Carbowax 400). Die Aushärtung erfolgt bei Zugabe von 80-100 % Wasser und einem Metallcarboxylat (Kaliumoctoat) als Katalysator wobei sich ein Schaum bildet, der mit Silikonöl stabilisiert wird.

Auf aliphatischen Isocyanaten basierte Systeme zeigen eine nur ungenügende Reaktivität und damit eine zu langsame Aushärtungszeit. Durch den Einsatz von Metallkatalysatoren konnte, wie in EP-A 0 482 467 beschrieben, zwar die Reaktionsgeschwindigkeit erhöht werden, doch kam es zur Bildung eines Schaumes, mit den oben beschriebenen Problemen.

Die Kombination von Asparaginsäureestem zur Vernetzung von Prepolymeren unter Ausbildung eines starken Gewebeklebstoffes wird in den nicht vorveröffentlichten Europäischen Patentanmeldungen Nr. 08012901.8, 08004134.6, 08001290.9 und 07012984.6 bereits beschrieben. Alternative Verbindungen zur Aminhärtung der Prepolymere werden hingegen nicht genannt.

Die Ausrüstung von Gewebeklebstoffen mit Wirkstoffen ist für verschiedene Gebiete interessant. Durch Verwendung von Analgetika wird das Schmerzempfinden an der zu behandelnden Stelle herabgesetzt oder ausgeschaltet, wodurch auf eine subcutane Injektion eines Analgetikums verzichtet werden kann. Besonders im Bereich der Tiermedizin, in der bei topischen Schnitten wie Kastrationen oder dem Mulesing bei Schafen nur selten Schmerzmittel eingesetzt werden, ist ein im Klebstoff integriertes Analgetikum indiziert. Durch Herabsetzung des Schmerzempfindens wird zudem die Gefahr eines traumatischen Schocks reduziert.

Die Verwendung von antimikrobiel/antiseptisch wirksamen Substanzen verhindert ein Eindringen von Keimen in die Wunde bzw. bewirkt ein Abtöten bereits vorhandener Bakterien. Dies ist speziell in der Tiermedizin von Interesse, da hier nur in seltenen Fällen aseptisch gearbeitet werden kann. Das gleiche gilt für antimykotisch wirkende Verbindungen zur Behandlung von Pilzinfektionen.

Allgemein versteht man unter pharmakologisch aktiven Verbindungen Stoffe und Zubereitungen aus Stoffen, die zur Anwendung am oder im menschlichen oder tierischen Körper bestimmt sind, um Krankheiten, Leiden, Körperschäden oder krankhafte Beschwerden zu heilen, zu lindern, zu verhüten oder zu erkennen. Darunter fallen ebenfalls Stoffe und Zubereitungen daraus, um Krankheitserreger, Parasiten oder körperfremde Stoffe abzuwehren, zu beseitigen oder unschädlich zu machen.

Ein Gewebeklebstoff sollte:
- eine starke Bindung zum Gewebe ausbilden
- einen transparenten Film bilden
- eine flexible Fügenaht formen
- durch eine eingestellte Viskosität leicht applizierbar sein und nicht in tiefere Gewebeschichten eindringen
- je nach Anwendungsgebiet eine Aushärtungszeit von wenigen Sekunden bis zu 10 Minuten haben
- keine wesentliche Exothermie beim Aushärten zeigen
- biokompatibel sein und keine Zell- und Gewebetoxizität zeigen

Unter Gewebe im Rahmen der vorliegenden Erfindung werden Zellverbände verstanden, die aus Zellen gleicher Gestalt und Leistung bestehen wie Deckgewebe (Haut), Epithelgewebe, Myocard, Binde- oder Stützgewebe, Muskeln, Nerven und Knorpel. Hierzu gehören unter anderem auch alle aus Zellverbänden aufgebaute Organe wie Leber, Niere, Lunge, Herz etc.

Es wurde nun gefunden, dass durch eine Kombination von isocyanatfunktionellen Prepolymeren auf Basis aliphatischer Isocyanate, wie denen der nicht vorveröffentlichten Europäischen Patentanmeldungen Nr. 08012901.8, 08004134.6, 08001290.9 und 07012984.6, mit speziellen strukturell von Aminosäuren abgeleiteten sekundären Diaminen Gewebeklebstoffe hergestellt werden können, die die oben genannten Bedingungen ebenfalls erfüllen.

Gegenstand der vorliegenden Erfindung sind daher Klebstoffsysteme umfassend
A) isocyanatfunktionelle Prepolymere erhältlich aus
   A1) aliphatischen Isocyanaten und
   A2) Polyolen mit zahlenmittleren Molekulargewichten von ≥400 g/mol und mittleren OH-Funktionalitäten von 2 bis 6
B1) sekundären Diaminen der allgemeinen Formel (I) 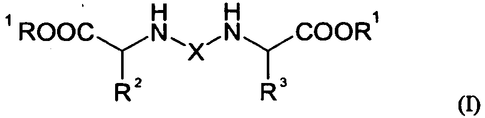 wobei
   - X: ein zweiwertiger gegebenenfalls heteroatomhaltiger Kohlenwasserstoffrest ist,
   - R¹: unabhängig voneinander gleiche oder verschiedene organische Reste sind, die keinen Zerewitinoff-aktiven Wasserstoff aufweisen und
   - R², R³: unabhängig voneinander gegebenenfalls substituierte und/oder heteroatomhaltige Kohlenwasserstoffreste mit 1 bis 9 Kohlenstoffatomen oder Wasserstoff sind,
B2) gegebenenfalls organische Füllstoffe, die eine nach DIN 53019 gemessenen Viskosität bei 23°C im Bereich von 10 bis 6000 mPas aufweisen und
C) gegebenenfalls eine oder mehrere pharmakologisch aktive Verbindungen.

Gemäß einer bevorzugten Ausführungsform der Erfindung haben R² und/oder R³ die oben genannte Bedeutung, sind aber nicht CH₂-COOR¹.

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Die in A) eingesetzten isocyanatfunktionellen Prepolymere sind durch Umsetzung von Isocyanaten mit hydroxyfunktionellen Polyolen gegebenenfalls unter Zusatz von Katalysatoren sowie Hilfs- und Zusatzstoffen erhältlich.

In A1) können als Isocyanate beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methan oder deren Mischungen beliebigen Isomerengehalts, 1,4-Cyclohexylendlisocyanat, 4-Isocyanatomethyl-1,8-octandiisocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret, Iminooxadiazindion- oder Oxadiazintrionstruktur sowie deren Mischungen eingesetzt werden.

Bevorzugt werden in A1) Isocyanate der vorstehend genannten Art mit ausschließlich aliphatisch oder cycloaliphatisch gebundenen Isocyanatgruppen oder deren Mischungen eingesetzt.

Die in A1) eingesetzten Isocyanate oder Isocyanatmischungen haben bevorzugt eine mittlere NCO-Funktionalität von 2 bis 4, besonders bevorzugt 2 bis 2,6 und ganz besonders bevorzugt 2 bis 2,4.

In einer besonders bevorzugten Ausführungsform wird in A1) Hexamethylendiisocyanat eingesetzt.

Zum Prepolymeraufbau können in A2) grundsätzlich alle dem Fachmann an sich bekannten Polyhydroxyverbindungen mit 2 oder mehr OH-Funktionen pro Molekül eingesetzt werden. Dies können beispielsweise Polyesterpolyole, Polyacrylatpolyole, Polyurethanpolyole, Polycarbonatpolyole, Polyetherpolyole, Polyesterpolyacrylatpolyole, Polyurethanpolyacryl-atpolyole, Polyurethanpolyesterpolyole, Polyurethanpolyetherpolyole, Polyurethanpolycarbonat-polyole, Polyesterpolycarbonatpolyole oder deren beliebige Mischungen untereinander sein.

Die in A2) eingesetzten Polyole haben bevorzugt eine mittlere OH-Funktionalität von 3 bis 4.

Die in A2) eingesetzten Polyole haben ferner bevorzugt ein zahlenmittleres Molekulargewicht von 400 bis 20000 g/mol, besonders bevorzugt 2000 bis 10000 g/mol und ganz besonders bevorzugt 4000 bis 8500 g/mol.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugte Polyalkylenoxid-Polyether entsprechen denen der vorstehend genannten Art und weisen einen Gehalt an Ethylenoxid-basierten Einheiten von 50 bis 100 mol%, bevorzugt von 60 bis 90 mol% und ganz besonders bevorzugt 70 bis 80 mol% bezogen auf die insgesamt enthaltene Menge an Alkylenoxideinheiten auf.

Bevorzugte Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiyl(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können hydroxylgruppenaufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt werden zum Prepolymeraufbau Polyetherpolyole der vorstehend genannten Art eingesetzt.

Zur Herstellung des Prepolymers werden die Verbindungen der Komponente A1) mit denen der Komponente A2) bei einem NCO/OH-Verhältnis von bevorzugt 4:1 bis 12:1, besonders bevorzugt 8:1 umgesetzt und anschließend der Anteil an nicht umgesetzten Verbindungen der Komponente A1) mittels geeigneter Methoden abgetrennt. Üblicherweise wird hierfür die Dünnschichtdestillation verwendet, wobei restmonomerenarme Produkte mit Restmonomergehalten von weniger als 1 Gew.-%, bevorzugt weniger als 0,5 Gew.-%, ganz besonders bevorzugt weniger als 0,1 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur beträgt dabei 20 bis 120°C, bevorzugt 60 bis 100°C.

X in Formel (I) kann ein zweiwertiger aliphatischer oder cycloaliphatischer Kohlenwasserstoffrest sein, welche in der C-C-Kette Heteroatome wie Sauerstoff, Schwefel oder substituierten/unsubstituierten Stickstoff tragen kann. Eine Substitution am Stickstoff kann eine Alkylgruppe, bevorzugt Methyl, Ethyl oder Propyl sein. Bevorzugt ist X in Formel (I) eine Alkylkette mit 4 bis 7 Kohlenstoffatomen.

R² und R³ leiten sich bevorzugt von natürlichen Aminosäuren der allgemeinen Formel R²-CH(NH₂)-COOH bzw. R³-CH(NH₂)-COOH aus der Gruppe Alanin, Leucin, Valin, t-Leucin, Isoleucin, Phenylalanin, Dihydroxyphenylalanin (Dopa), Tyrosin, Histidin, Methionin, Prolin, Arginin, Asparagin, Asparaginsäure, Cystein, Glutaminsäure, Glutamin, Lysin, Serin und Threonin ab.

Besonders bevorzugt sind R² und R³ damit unabhängig voneinander -CH₃, -CH₂CH(CH₃)₂, - CH(CH₃)₂, -C(CH₃)₃, -CH(CH₃)CH₂CH₃, Phenyl-, 2,3-Dihydroxyphenyl-, Alkyl- oder Cycloalkylreste mit 1 bis 9, bevorzugt 1 bis 4 C-Atomen, die in der Kette oder endständig ein Heteroatom der Gruppe Schwefel, Sauerstoff und Stickstoff gegebenenfalls als Teil einer funktionellen Gruppe aufweisen. Endständige Hydroxy- Amino- und Carboxyfunktionen können selbstverständlich auch alkyliert sein.

Ganz besonders bevorzugt sind R² und R³ unabhängig voneinander -CH₂CH(CH₃)₂, -CH(CH₃)₂, - C(CH₃)₃, -CH(CH₃)CH₂CH₃.

Grundsätzlich können R² und R³ im Rahmen der vorgenannten Bereiche unabhängig voneinander variieren, wobei bevorzugt jedoch R² = R³ ist.

Grundsätzlich ist für das Funktionieren der vorliegenden Erfindung die Konfiguration am Stereozentrums α-ständig zur Amino- bzw. R²/R³-Gruppe unerheblich. Dienen zur Herstellung der sekundären Diamine der Formel (I) Aminosäuren bzw. deren Ester als Ausgangsmaterialien, so können diese jeweils enantio merenrein oder als racemische Gemische verwendet werden.

R' ist bevorzugt ein C₁- bis C₁₀-Alkylrest, besonders bevorzugt Methyl oder Ethyl.

In einer bevorzugten Ausführungsform der Erfindung ist R¹ = Methyl, wobei X auf 1,5-Diaminopentan als n-wertigem Amin basiert.

Die Herstellung der sekundären Diamine der Komponente B1) kann zum Beispiel in bekannter Weise durch reduktive Aminierung des entsprechenden Oxoacetates mit einem primären difunktionellen Amin erfolgen (Gleichung 1).

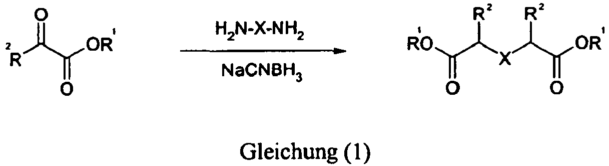

Bevorzugte primäre difunktionelle Amine X(NH₂)₂ sind Ethylendiamin, 1,2-Diaminopropan, 1,4-Diaminobutan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexyl-methan, 2,4,4'-Triamino-5-methyl-dicyclohexylmethan und Polyetheramine mit aliphatisch gebundenen primären Aminogruppen mit einem zahlenmittleren Molekulargewicht Mₙ von 148 bis 6000 g/mol.

Besonders bevorzugte primäre difunktionelle Amine sind 1,3-Diaminopropan, 1,3-Diaminobulan, 1,5- Diaminopentan und 1,6-Diaminohexan.

Ebenso kann die Herstellung durch Umsetzung des geschützten Aminosäureesters mit dem entsprechenden Dialdehyd über das Diimin und anschließender Entschützung erfolgen (Gleichung 2).

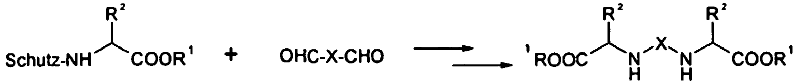

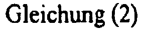

Die Produkte können je nach Kettenlänge x auch durch Umsetzung des entsprechenden Aminosäureesterhydrochlorides mit einer Dibromalkylverbindung erhalten werden:

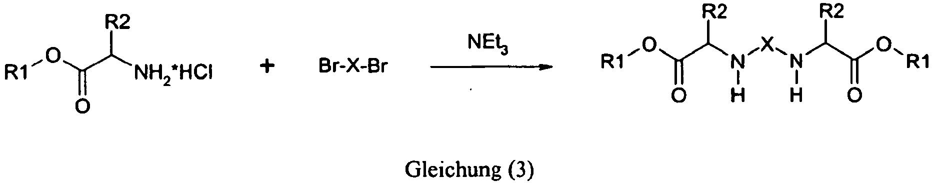

Die Reaktion kann ebenfalls so durchgeführt werden, dass ein unsymmetrisches Endprodukt entsteht (Gleichung 4):

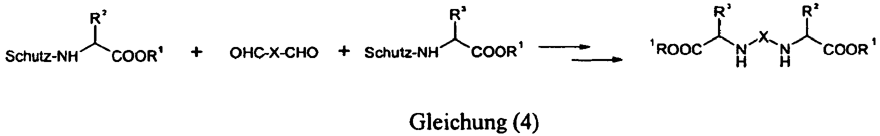

In Gleichung (2, 3 und 4) werden als Edukte natürliche und nicht- natürliche Aminosäureester verwendet. Um eine intramolekulare Cyclisierung zu verhindern, werden die Aminosäureester N-terminal geschützt. Als Schutzgruppen können alle geeigneten, dem Chemiker bekannten Systeme verwendet werden (z. B. tert.-Butoxycarbonyl (Boc), Benzyloxycarbonyl (Z)).

X kann bei Verwendung der Dialdehyde bzw. Dibromide OHC-X-CHO bzw. Br-X-Br eine Alkylkette mit 2 bis 6, bevorzugt 2 oder 3 Kohlenstoffatomen sein.

Die in B2) eingesetzten organischen flüssigen Füllstoffe sind bevorzugt gemäß Zytotoxizitätsmessung nach ISO 10993 nicht zytotoxisch.

Beispielsweise können als organische Füllstoffe flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Bevorzugt handelt es sich bei den organischen Füllstoffen der Komponente B2) um hydroxyfunktionelle Verbindungen. Bevorzugte hydroxyfunktionelle Verbindungen sind Polyether und/oder Polyesterpolyole, besonders bevorzugt Polyetherpolyole.

Die bevorzugten organischen Füllstoffe der Komponente B2) besitzen bevorzugt mittlere OH-Funktionalitäten von 1,5 bis 3, besonders bevorzugt 1,8 bis 2,2, ganz besonders bevorzugt 2,0.

Die bevorzugten organischen Füllstoffe der Komponente B2) besitzen bevorzugt sich wiederholende von Ethylenoxid abgeleitete Einheiten.

Die Viskosität der organischen Füllstoffe der Komponente B2) beträgt bevorzugt 50 bis 4000 mPas bei 23°C gemessen nach DIN 53019.

In einer bevorzugten Ausführungsform der Erfindung werden als organische Füllstoffe der Komponente B2) Polyethylenglycole eingesetzt. Diese haben bevorzugt ein zahlenmittleres Molekulargewicht von 100 bis 1000 g/mol, besonders bevorzugt 200 bis 400 g/mol.

Das Gewichtsverhältnis von B1) zu B2) beträgt 1:0 bis 1:20 , bevorzugt 1:0 bis 1:12.

Das Gewichtsverhältnis der Komponente B2) bezogen auf die Gesamtmenge der Mischung aus B 1, B2 und A liegt im Bereich von 0 bis 100 %, bevorzugt 0 bis 60 %.

Pharmakologisch aktive Wirkstoffe können unter anderem aber nicht ausschließlich sein:
a) Analgetika mit und ohne antiinflammatorische Wirkung
b) Antiphlogistika
c) Antimikrobiell wirksame Substanzen
d) Antimykotika
e) Antiparasitär wirkende Stoffe

Der Wirkstoff ist bevorzugt bei Raumtemperatur in der Härterkomponente B1) löslich, kann aber auch in B1) suspendiert eingesetzt werden. In einer bevorzugten Ausführungsform der Erfindung wird der Wirkstoff in einer Mischung aus Härter B1) und Füllstoff B2) gelöst oder suspendiert, wobei als B2) bevorzugt Polyethylenglycole mit einem zahlenmittleren Molekulargewicht von 100 bis 1000 g/mol, besonders bevorzugt von 200 bis 400 g/mol eingesetzt werden.

Die Konzentration des zugesetzten Wirkstoffes richtet sich nach den therapeutisch notwendigen Dosen und liegt bei 0,001 Gew.-% bis 10 Gew.-%, bevorzugt bei 0,01 Gew.-% bis 5 Gew.-% bezogen auf die Gesamtmenge aller nichtflüchtigen Komponenten des Klebstoffsystems

Alle einsetzbaren Wirkstoffe zeichnen sich dadurch aus, dass sie nicht über NCO-reaktive funktionelle Gruppen verfügen, bzw. dass die Reaktion eventuell vorhandener funktioneller Gruppen mit dem Isocyanat-Prepolymer gegenüber der Diamin- NCO-Reaktion deutlich langsamer ist.

Analgetika, die diesen Anspruch erfüllen, sind Lokalanästhetika wie Ambucain, Amylocain, Arecaidin, Benoxinat, Benzocain, Betoxycain, Butacain, Butethamin, Bupivacain, Butoxycain, Chlorprocain, Cocaethylen, Cocaine, Cyclomethycain, Dibucain, Dimethocain, Dimethisoquin, Etidocain, Fomocain, Isobutyl-p-aminobenzoat, Leucinocain, Lidocain, Meperidin, Mepivacain, Metabutoxycain, Octacain, Orthocain, Oxethazain, Phenacain, Piperocain, Piridocain, Pramoxin, Procain, Procainamid, Proparacain, Propoxycain, Pseudococain, Pyrrocain, Ropivacain, Tetracain, Tolycain, Tricain, Trimecain, Tropacocain, Amolanon, Cinnamoylcocain, Parethoxycain, Propiocain, Myrtecain und Propanocain.

Ebenso eingesetzt werden können Opioid-Analgetika wie Morphin und seine Derivate (z. B. Codein, Diamorphin, Dihydrocodein, Hydromorphon, Oxycodon, Hydrocodon, Buprenorphin, Nalbuphin, Pentazocin), Pethidin, Levomethadon, Tilidin und Tramadol.

Gleichfalls können nichtsteroidale Antiphlogistika (NSAID) wie Acetylsalicylsäure, Acemetacin, Dexketoprofen, Diclofenac, Aceclofenac, Diflunisal, Piritramid, Etofenamat, Felbinac, Flurbiprofen, Flufenaminsäure, Ibuprofen, Indometacin, Ketoprofen, Lonazolac, Lornoxicam, Mefenaminsäure, Meloxicam, Naproxen, Piroxicam, Tiaprofensäure, Tenoxicam, Phenylbutazon, Propyphenazon, Phenazon und Etoricoxib verwendet werden. Andere Analgetika wie Azapropazon, Metamizol, Nabumeton, Nefopam, Oxacephrol, Paracetamol sowie das analgetisch wirksame Amitriptylin können selbstverständlich ebenfalls eingesetzt werden.

Neben den genannten Analgetika, die eine entzündungshemmende Wirkung haben, können zusätzlich rein antiphlogistisch wirksame Verbindungen eingesetzt werden. Dazu gehört die Klasse der Glucocorticoide wie z. B. Cortison, Betamethason, Dexamethason, Hydrocortison, Methylprednisolon, Prednisolon, Prednison, Budesonid, Allotetrahydro-cortison, Fludrocortison, Fluprednisolon, Fluticasonpropionat, etc.

Als antiseptisch wirksame Substanzen können unter anderem die folgenden Verbindungen eingesetzt werden: Triclosan (2,4,4'-Trichloro-2'hydroxydiphenyl ether), Chlorhexidin und seine Salze, Octenidin, Chloramphenicol, Florfenicol, Chlorchinaldol, Iod, Povidon-Iod, Hexachlorophen, Merbromin, PHMB, nanokristallin vorliegendes Silber sowie Silber- und Kupfersalze.

Des weiteren können als antimikrobiell wirksame Substanzen Antibiotika aus der Klasse der β-Lactame (z. B. Penicillin und seine Derivate, Cephalosporine), der Tetracycline (z. B. Demeclocyclin, Doxycyclin, Oxytetracycline, Minocyclin, Tetracyclin), der Makrolide (z. B. Erythromycin, Josamycin, Spiramycin), der Lincosamide (z. B. Clindamycin, Lincomycin), der Oxazolidinone (z. B. Linezolid), der Gyrasehemmer (z. B. Danofloxacin, Difloxacin, Enrofloxacin, Ibafloxacin, Marbofloxacin, Nalidixinsäure, Pefloxacin, Fleroxacin, Levofloxacin) und der zyklischen Peptide (z. B. Bicozamycin) verwendet werden. Ebenso eingesetzt werden können Rifamycin, Rifaximin, Methenamin; Mupirocin, Fusidinsäure, Flumechin, und die Derivate des Nitroimidazols (z. B. Metronidazol, Nimorazol, Tinidazol), des Nitrofurans (Furaltadon, Nifurpirinol, Nihydrazone, Nitrofurantoin), des Sulfonamids (z. B. Sulfabromomethazin, Sulfacetamid, Sulfachlorpyridazine, Sulfadiazine etc.) sowie β-Lactamase-Inhibitoren wie die Clavulansäure.

Als antimykotisch wirksame Substanzen können alle Azolderivate, die die Biosynthese von Ergosterol hemmen, wie z. B. Clotrimazol, Fluconazol, Miconazol, Bifonazol, Econazol, Fenticonazol, Isoconazol, Oxiconazol etc. eingesetzt werden. Sonstige lokal anwendbare Antimykotika sind Amorolfin, Ciclopirox, Thymol und seine Derivate sowie Naftifin. Ebenso kann die Klasse der Alkylparabene eingesetzt werden.

Zu den antiparasitär wirksamen Verbindungen gehören unter anderem die Ektoparasitizide Cyfluthrin und Lindan, verschiedene Azolderivate wie z. B. Dimetridazol und Metronidazol sowie Chinin.

Bei Bedarf kann die Härterkomponente angefärbt werden.

Die erfindungsgemäßen Klebstoffsysteme werden durch Mischung des Prepolymers A mit dem sekundären Diamin der Komponente B) erhalten. Das Verhältnis von Aminogruppen zu freien NCO-Gruppen beträgt bevorzugt 1:1,5 bis 1:1, besonders bevorzugt 1:1.

Die erfindungsgemäßen Klebstoffsysteme besitzen unmittelbar nach Vermischung der Einzelkomponenten miteinander eine Scherviskosität bei 23°C von bevorzugt 1000 bis 10000 mPas, besonders bevorzugt 2000 bis 8000 mPas und ganz besonders bevorzugt 2500 bis 5000 mPas.

Die Zeit bei 23°C bis eine Aushärtung des Klebestoffs ohne Klebrigkeit der Oberfläche erreicht ist, beträgt typischerweise 30 s bis 10 min, bevorzugt 1 min bis 8 min, besonders bevorzugt 1 min bis 5 min.

Ein weiterer Gegenstand der Erfindung sind die aus den erfindungsgemäßen Klebstoffsystemen erhältlichen Klebfilme sowie daraus hergestellte Verbundteile.

In einer bevorzugten Ausführungsform werden die erfindungsgemäßen Klebstoffsysteme als Gewebekleber für den Verschluss von Wunden in menschlichen oder tierischen Zellverbänden verwendet, so dass auf ein Klammern oder Nähen zum Verschließen weitestgehend verzichtet werden kann.

Der erfindungsgemäße Gewebekleber kann so wohl *in vivo* als auch *in vitro* angewendet werden, wobei die *in vivo* Anwendung beispielsweise zur Wundbehandlung nach Unfällen oder Operationen bevorzugt ist.

Daher ist auch ein Gegenstand der vorliegenden Erfindung ein Verfahren zum Verschließen oder Verbinden von Zellgeweben, dadurch gekennzeichnet, dass die erfindungsgemäßen Klebstoffsysteme eingesetzt werden.

Ebenfalls Gegenstand der Erfindung ist ferner die Verwendung solcher Klebstoffsysteme zur Herstellung eines Mittels zum Verschließen oder Verbinden von Zellgeweben, so wie die zur Applikation notwendigen 2-Kammerdosiersysteme umfassend die erfindungswesentlichen Komponenten des Klebstoffsystems.

### Beispiele:

Sofern nicht abweichend angegeben beziehen sich alle Prozentangaben auf das Gewicht.

Als Gewebeersatz wurde Rindfleisch verwendet. Es wurden jeweils zwei Stücke Fleisch (1 = 4 cm, h = 0.3 cm, b = 1 cm) an den Enden 1 cm weit mit dem Kleber bestrichen und überlappend geklebt. Die Stabilität der Klebeschicht wurde jeweils durch Zug überprüft.

### Beispiel 1, (Prepolymer A-1)

465 g HDI und 2.35 g Benzoylchlorid wurden in einem 11 Vierhalskolben vorgelegt. Innerhalb von 2h wurden bei 80°C 931.8 g eines Polyethers mit einem Ethylenoxidgehalt von 71 % und einem Propylenoxidgehalt von 29 % (jeweils bezogen auf dem gesamten Alkylenoxidgehalt) gestartet auf TMP (3-funktionell) hinzugefügt und rührte 1h nach. Anschließend wurde durch Dünnschichtdestillation bei 130°C und 0,1 Torr das überschüssige HDI abdestilliert. Man erhielt 980 g (71 %) des Präpolymers mit einem NCO-Gehalt von 2,53%. Der Restmonomerengehalt betrug < 0,03 % HDI.

### Beispiel 2, (Dimethyl 2,2'-(pentan-1,5-diylbis(azandiyl))bis(4-methylpentanoat)) (1)

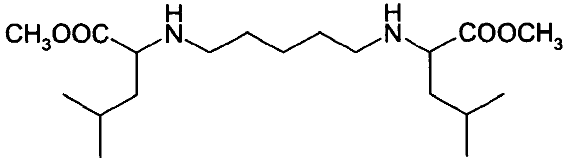

2 Mol Z- geschützter Leucinmethylester wurde mit 1 Mol Glutardialdehyd unter dreitägigem Rühren in Methanol bei Raumtemperatur zum Diimin umgesetzt. Dieses wurde anschließend auf Pd/C in Methanol hydriert. Das Produkt wurde säulenchromatographisch gereinigt.

¹H-NMR (CDCl₃, 400 MHz): δ = 0.91 (d, 6H), 0.94 (d, 6H), 1.35 (m, 2H), 1.48 (m, 8H), 1.69 (m, 2H), 2.43 (m, 2H), 2.56 (m, 2H), 3.29 (t, 2H), 3.72 (s, 6H).
¹³C-NM-R (CDCl₃, 400 MHz): δ = 22.3, 22.5, 24.7, 24.8, 29.9, 42.7, 48.0, 51.4, 59.9, 176.5

### Beispiel 3, (Diethyl 2.2'-(pentan-1,5-diyldiimino)dipropanoat) (2)

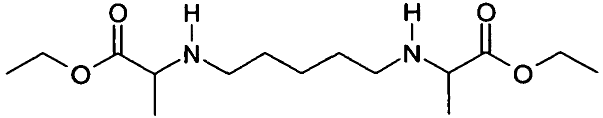

5 g Brenztraubensäureethylester (2 eq) wurden in 100 ml absolutem Ethanol gelöst und mit 15.9 g 1,5-Diaminopentan versetzt. Unter Eiskühlung wurden 21.2 g (2 eq) Natriumcyanoborhydrid zugesetzt. Man rührt über Nacht bei Raumtemperatur nach. Nach Hydrolyse wird das Produkt mit Methylenchlorid ausgeschüttelt. Anschließend erfolgt eine säulenchromatographische Reinigung (Methanol/Ethylacetat 1:6). Es wurden 3 g des Produktes als gelbe Flüssigkeit erhalten

¹H-NMR (CDCl₃, 400 MHz): δ = 1.11 (d, 6H), 1.18 (t, 6H), 1.22 (m, 2H), 1.38 (m, 4H), 2.38 (m, 2H), 2.48 (m, 2H), 3,22 (m, 2H), 4.08 (q, 4H).
¹³C-NMR (CDCl₃, 400 MHz): δ = 14.2, 18.8, 24.8, 29.6, 47.7, 56.6, 61.4, 174.6

### Beispiel 4, (Diethyl 2,2'-(butan-1,4-diyldiimino)dipropanoat (3)

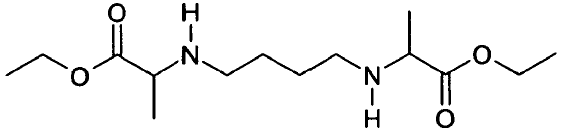

Analog zu Beispiel 2 wurden aus 5 g Brenztraubensäureethylester und 13.7 g 1,4-Diaminobutan 3.5 g des Produktes als gelbe Flüssigkeit erhalten.

¹H-NMR (CDCl₃, 400 MHz): δ = 1.30 (d, 6H), 1.31 (t, 6H), 1.58 (m, 4H), 2.50 (m, 2H), 2.60 (m, 2H), 3,41 (q, 2H), 4.21 (q, 4H).
¹³C-NMR (CDCl₃, 400 MHz): δ = 14.2, 18.4, 27.5, 47.5, 56.6, 60.4, 175.4.

### Beispiel 5, (Dimethyl-2,2'-(propan-1,3-diyldiimino)bis(3-methylbutanoat) (4)

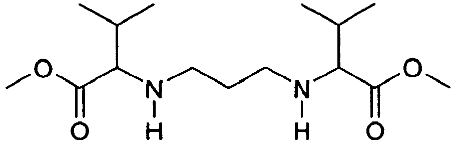

5.36 g 1,3-Dibrompropan (1 eq) wurden in 25 ml Methanol gelöst und mit 10.74 g (2 eq) Triethylamin versetzt. Anschließend wurden 8.9 g (1 eq) 1-Valinmethylester-Hydrochlorid zugesetzt. Die Reaktionsmischung wurde fünf Tage zum Rückfluß erhitzt. Nach Abkühlen auf Raumtemperatur wurde das Gemisch in Dichlormethan aufgenommen und mehrfach mit Wasser extrahiert. Nach Trocknen über Magnesiumsulfat wurde das Lösemittel im Vakuum entfernt. Es wurden 4.2 g des Produktes als gelbe Flüssigkeit erhalten.

¹H-NMR (CDCl₃, 400 MHz): δ = 0.90 (d, 6H), 0.96 (d, 6H), 1.67 (m, 2H), 1.92 (m, 2H), 2.50 (m, 2H), 2.68 (m, 2H), 2.98 (d, 2H), 3.71 (s, 6H).
¹³C-NMR (CDCl₃, 400 MHz): δ = 18.7, 19.2, 19.7, 31.4, 46.6, 51.1, 67.4, 175.4.

### Beispiel 6, (Dimethyl-2,2'-(propan-1,3-diyldiimino)bis(3-methylpentanoat) (5)

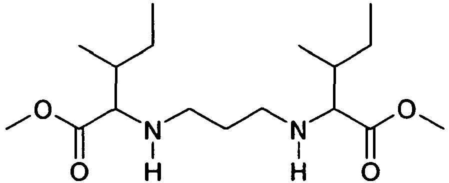

Analog zu Beispiel 4 wurden aus 5.2 g 1,3-Dibrompropan, 10.43 g Triethylamin und 9.36 g L-Isoleucinmethylester-Hydrochlorid 4.5 g des Produktes als gelbe Flüssigkeit hergestellt.

¹H-NMR (CDCl₃, 400 MHz): δ = 0.89 (d, 6H), 0.94 (d, 6H), 1.20 (m, 2H), 1.61 (m, 6H), 2.49 (m, 2H), 2.68 (m, 2H), 3.02 (d, 2H), 3.71 (s, 6H).
¹³C-NMR (CDCl₃, 400 MHz): δ = 11.4, 11.7, 15.6, 25.7, 38.4, 47.3, 51.3, 66.2, 175.6.

### Beispiel 7, (Dimethyl-2,2'-(propan-1,3-diyldiimino)bis(3-phenylpropanoat) (6)

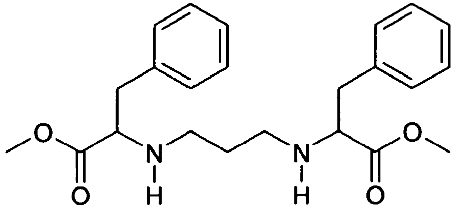

Analog zu Beispiel 4 wurden aus 4.86 g 1,3-Dibrompropan, 9.75 g Triethylamin und 10.39 g L-Phenylalaninmethylester-Hydrochlorid 3.8 g des Produktes als gelbe Flüssigkeit nach säulenchromatographischer Reinigung (Methanol/Ethylacetat 1:6) erhalten.

¹H-NMR (CDCl₃, 400 MHz): δ = 1.59 (m, 2H), 2.50 (m, 2H), 2.62 (m, 2H), 2.89 (m, 4H), 3.43 (d, 2H), 3.61 (s, 6H), 7.22 (m, 10H).
¹³C-NMR (CDCl₃, 400 MHz): δ = 41.1, 46.5, 51.5, 62.8, 63.2, 126.1, 128.3, 129.2, 137.3, 174.9.

### Beispiel 8, (Dimethyl-2,2'-(hexan-1,6-diyldiimino)bis(3-phenylpropanoat) (6)

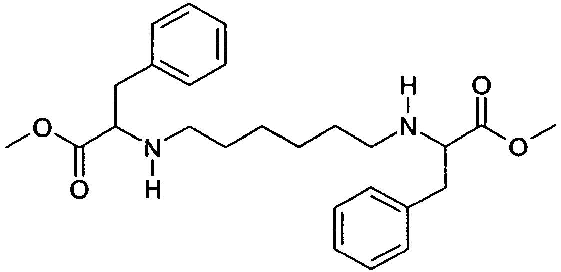

Analog zu Beispiel 4 wurden aus 5.65 g 1,6-Dibromhexan, 9.37 g Triethylamin und 9.99 g L-Phenylalaninmethylester-Hydrochlorid 3.1 g des Produktes als gelbe Flüssigkeit nach säulenchromatographischer Reinigung (Methanol/Ethylacetat 1:6) erhalten.

¹H-NMR (CDCl₃, 400 MHz): δ = 1.22 (m, 2H), 1.54 (m, 4H), 2.42 (m, 2H), 2.58 (m, 2H), 2.68 (m, 2H), 2.90 (m, 4H), 3.48 (d, 2H), 3.69 (s, 6H), 7.19 (m, 10H).
¹³C-NMR (CDCl₃, 400 MHz): δ = 29.9, 36,5, 39.6, 51.4, 63,9, 69,8, 126.0, 128.3, 129.1, 138.8, 174.5.

### Beispiel 9, (Gewebekleber)

1 g des Prepolymers A-1 wurden mit einer equivalenten Menge Dimethyl 2,2'-(pentane-1,5-diylbis(azandiyl))bis(4-methylpentanoate) in einem Becher gut verrührt. Die Reaktionsmischung wurde unmittelbar danach auf das zu klebende Gewebe dünn aufgetragen. Eine Aushärtung zu einem durchsichtigen Film mit einer damit verbundenen starken Klebung hatte innerhalb von 1 min stattgefunden. Die Oberfläche des Klebstoffes war nach 2 min nicht mehr klebrig.

| **Verbindung** | **Verarbeitungszeit** | **Klebestärke** |
|---|---|---|
| 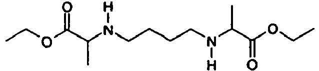 | 42 s | - |
| 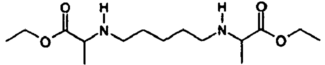 | 33 s | - |
| 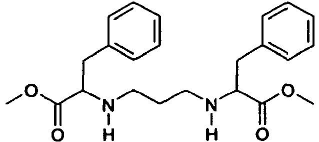 | 4 min | - |
| 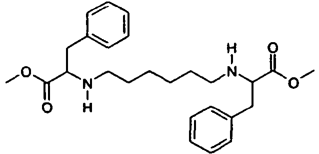 | 4 min | - |
| 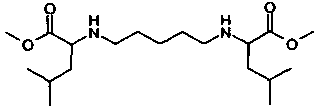 | 4 min | ++ |
| 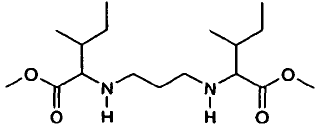 | 3 min | ++ |
| 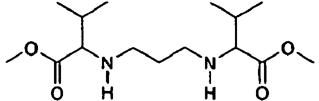 | 4 min | ++ |

| | | |
|---|---|---|
| ++: starke Klebekraft, das Gewebe zerreißt bei Zug -: schwache Klebekraft, die Klebenaht löst sich vom Gewebe ab | | |

## Patentansprüche

1. Klebstoffsysteme umfassend
A) isocyanatfunktionelle Prepolymere erhältlich aus
A1)aliphatischen Isocyanaten und
A2) Polyolen mit zahlenmittleren Molekulargewichten von ≥400 g/mol und mittleren OH-Furktionalitäten von 2 bis 6
B1) sekundären Diaminen der allgemeinen Formel (I) 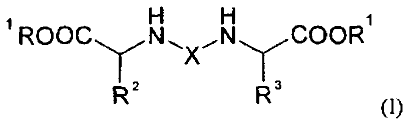 wobei
X ein zweiwertiger gegebenenfalls heteroatomhaltiger Kohlenwasserstoffrest ist,
R¹ unabhängig voneinander gleiche oder verschiedene organische Reste sind, die keinen Zerewitinoff-aktiven Wasserstoff aufweisen und
R²R³ unabhängig voneinander gegebenenfalls substituierte und/oder heteroatomhaltige Kohlenwasserstoffreste mit 1 bis 9 Kohlenstoffatomen oder Wasserstoff sind,
B2) gegebenenfalls organische Füllstoffe, die eine nach DIN 53019 gemessenen Viskosität bei 23°C im Bereich von 10 bis 6000 mPas aufweisen und
C) gegebenenfalls eine oder mehrere pharmakologisch aktive Verbindungen,
**dadurch gekennzeichnet, dass** es sich um einen Gewebekleber für menschliches oder tierisches Gewebe handelt.

2. Klebstoffsysteme gemäß Anspruch 1, **dadurch gekennzeichnet, dass** die in A1) eingesetzten Isocyanate ausschließlich aliphatisch oder cycloaliphatisch gebundene Isocyanatgruppen aufweisen.

3. Klebstoffsysteme gemäß Anspruch 1 oder 2, **dadurch gekennzeichnet, dass** die in A1) eingesetzten Isocyanate eine mittlere NCO-Funktionalität von 2 bis 2,4 aufweisen.

4. Klebstoffsysteme gemäß einem der Ansprüche 1 bis 3, **dadurch gekennzeichnet, dass** die in A2) eingesetzten Polyole zahlenmittlere Molekulargewichte von 4000 bis 8500 g/mol aufweisen.

5. Klebstoffsysteme gemäß einem der Ansprüche 1 bis 4, **dadurch gekennzeichnet, dass** die in A2) eingesetzten Polyole mittlere OH-Funktionalitäten von 3 bis 4 aufweisen

6. Klebstoffsysteme gemäß einem der Ansprüche 1 bis 5, **dadurch gekennzeichnet, dass** in A2) Polyalkylenoxid-Polyether eingesetzt werden.

7. Klebstoffsysteme gemäß Anspruch 6, **dadurch gekennzeichnet, dass** die in A2) eingesetzten Polyalkylenoxid-Polyether einen Gehalt an Ethylenoxid-basierten Einheiten von 60 bis 90 mol% bezogen auf die insgesamt enthaltene Mengen an Alkylenoxideinheiten aufweisen.

8. Klebstoffsysteme gemäß einem der Ansprüche 1 bis 7, **dadurch gekennzeichnet, dass** in Formel (I) X eine Alklkette mit 4 bis 7 Kohlenstoffatomen, R² und R³ unabhängig voneinander -CH₃, - CH₂CH(CH₃)₂, -CH(CH₃)₂, -C(CH₃)₃, -CH(CH₃)CH₂CH₃, Phenyl-, 2,3-Dihydroxyphenyl-, Alkyl- oder Cycloalkylreste mit 1 bis 9 C-Atome sind, die in der Kette oder endständig ein Heteroatom der Gruppe Schwefel, Sauerstoff und Stickstoff gegebenenfalls als Teil einer funktionellen Gruppe aufweisen , und R¹ ein C₁- bis C₁₀-Alkylrest ist.

9. Verwendung von Klebstoffsystemen gemäß einem der Ansprüche 1 bis 8 zur Herstellung eines Mittels zum Verschließen oder Verbinden von Zellgeweben.

## Claims

1. Adhesive system comprising
A) prepolymers with isocyanate groups, obtainable from
A1) aliphatic isocyanates and
A2) polyols with number average molecular weights of ≥ 400 g/mol and average OH group contents of 2 to 6
B1) secondary diamines of the general formula (I) 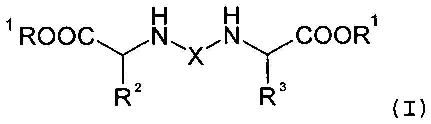 wherein
X is a divalent optionally heteroatom-containing hydrocarbon residue,
R¹ mutually independently are the same or different organic residues which have no Zerevitinov-active hydrogen and
R², R³ mutually independently are optionally substituted and/or heteroatom-containing hydrocarbon residues with 1 to 9 carbon atoms or hydrogen,
B2) optionally organic fillers, which exhibit a viscosity measured according to DIN 53019 at 23°C in the range from 10 to 6000 mPas and
C) optionally one or more pharmacologically active compounds,
**characterized in that** it is a tissue adhesive for human or animal tissue.

2. Adhesive system according to Claim 1, **characterized in that** the isocyanates used in A1) have exclusively aliphatically or cycloaliphatically bound isocyanate groups.

3. Adhesive system according to Claim 1 or 2, **characterized in that** the isocyanates used in A1) have an average NCO group content of 2 to 2.4.

4. Adhesive system according to one of Claims 1 to 3, **characterized in that** the polyols used in A2) have number average molecular weights of 4000 to 8500 g/mol.

5. Adhesive system according to one of Claims 1 to 4, **characterized in that** the polyols used in A2) have average OH group contents of 3 to 4.

6. Adhesive system according to one of Claims 1 to 5, **characterized in that** polyalkylene oxide polyethers are used in A2).

7. Adhesive system according to Claim 6, **characterized in that** the polyalkylene oxide polyethers used in A2) have a content of ethylene oxide-based units of 60 to 90 mol% based on the total quantities of alkylene oxide units contained.

8. Adhesive system according to one of Claims 1 to 7, **characterized in that** in formula (I) X is an alkyl chain with 4 to 7 carbon atoms, R² and R³ are mutually independently -CH₃, -CH₂CH(CH₃)_{2,} -CH(CH₃)₂, -C(CH₃)_{3,} - CH(CH₃)CH₂CH₃, phenyl, 2,3-dihydroxyphenyl, alkyl or cycloalkyl residues with 1 to 9 C atoms, which optionally have a heteroatom from the group sulphur, oxygen and nitrogen as part of a functional group in the chain or terminally, and R¹ is a C₁ to C₁₀ alkyl residue.

9. Use of adhesive systems according to one of Claims 1 to 8 for the production of a means for the closure or bonding of cell tissues.

## Revendications

1. Systèmes d'adhésif comprenant
A) des polymères à fonction isocyanate, pouvant être obtenus à partir
A1) d'isocyanates aliphatiques et
A2) de polyols ayant des masses moléculaires moyennes en nombre de ≥ 400 g/mole et des fonctionnalités OH moyennes de 2 à 6
B1) des diamines secondaires de formule générale (I) 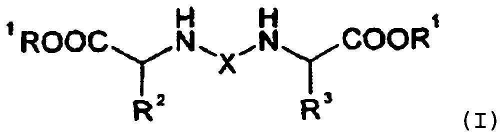 dans laquelle
X est un radical hydrocarboné divalent contenant éventuellement des hétéroatomes,
R¹ représentent indépendamment l'un de l'autre des radicaux organiques identiques ou différents, qui ne comportent pas d'hydrogène actif selon la méthode de Zerewitinoff et
R², R³ représentent indépendamment l'un de l'autre des radicaux hydrocarbonés éventuellement substitués et/ou contenant des hétéroatomes, ayant de 1 à 9 atomes de carbone, ou un atome d'hydrogène,
B2) éventuellement des charges organiques qui présentent une viscosité à 23 °C, mesurée selon DIN 53019, dans la plage de 10 à 6 000 mPa.s et
C) éventuellement un ou plusieurs composés pharmacologiquement actifs,
**caractérisés en ce qu'**il s'agit d'une colle à tissus pour tissu humain ou animal.

2. Systèmes d'adhésif selon la revendication 1, **caractérisés en ce que** les isocyanates utilisés en A1) comportent exclusivement des groupes isocyanate en liaison aliphatique ou cycloaliphatique.

3. Systèmes d'adhésif selon la revendication 1 ou 2, **caractérisés en ce que** les isocyanates utilisés en A1) présentent une fonctionnalité NCO moyenne de 2 à 2,4.

4. Systèmes d'adhésif selon l'une quelconque des revendications 1 à 3, **caractérisés en ce que** les polyols utilisés en A2) présentent des masses moléculaires moyennes en nombre de 4 000 à 8 500 g/mole.

5. Systèmes d'adhésif selon l'une quelconque des revendications 1 à 4, **caractérisés en ce que** les polyols utilisés en A2) présentent des fonctionnalités OH moyennes de 3 à 4.

6. Systèmes d'adhésif selon l'une quelconque des revendications 1 à 5, **caractérisés en ce qu'**en A2) on utilise des polyoxyalkylène-polyéthers.

7. Systèmes d'adhésif selon la revendication 6, **caractérisés en ce que** les polyoxyalkylène-polyéthers utilisés en A2) présentent une teneur en unités à base d'oxyde d'éthylène de 60 à 90 % en moles par rapport aux quantités contenues au total d'unités oxyde d'alkylène.

8. Systèmes d'adhésif selon l'une quelconque des revendications 1 à 7, **caractérisés en ce que** dans la formule (I) X représente un chaîne alkyle ayant de 4 à 7 atomes de carbone, R² et R³ représentent indépendamment l'un de l'autre -CH₃, -CH₂CH(CH₃)₂, -CH(CH₃)₂, -C(CH₃)₃, -CH(CH₃)CH₂CH₃, des radicaux phényle, 2,3-dihydroxyphényle, alkyle ou cycloalkyle ayant de 1 à 9 atomes de carbone, qui comportent dans la chaîne ou en bout de chaîne un hétéroatome choisi dans l'ensemble constitué par les atomes de soufre, d'oxygène et d'azote éventuellement en tant que partie d'un groupe fonctionnel, et R¹ est un radical alkyle en C₁-C₁₀.

9. Utilisation de systèmes d'adhésif selon l'une quelconque des revendications 1 à 8, pour la fabrication d'un produit destiné à la fermeture ou à la jonction de tissus cellulaires.
